# EUROPEAN PATENT APPLICATION

(11) **EP 0 849 388 A1**
(43) Date of publication of application: **24.06.1998**
(21) Application number: 96309212.7
(22) Date of filing: 17.12.1996
(51) Int. Cl.: D04H 13/00, A61F 13/15, A61F 13/00, B32B 5/06, B32B 5/26

(54) **Fabrication process for non-woven fabric and products thereof**

(71) Applicant: AM Medical Fabrics Limited, Hong Kong (HK)
(72) Inventor: Jeffcoat, Marilyn Olga, Hong Kong (HK)
(74) Representative: Luckhurst, Anthony Henry William

(57) **Abstract**

A process for the fabrication of non-woven fabric products able to transport lift and hold fluid or moisture by means of capillary action by using non-porous fibres capable of producing a non-woven fabric product made from two fibre batts of 100% polyester fibre, 1.2-1.5 denier, semi-dull, hollow or solid, needle-punched onto a "screen" (which is woven fabric made from any combination of polyester and cotton yarns of approximately 100 grams per sq m). The screen is positioned in the middle of the two non-woven fabric batts (each of approx 300 grams per sq m). One fibre batt is needle-punched onto one side of the screen and the other fibre batt onto the other side of the screen. Next the fabricated composite fibre batt should be run through the needleloom on both sides until the desired thickness or effect is obtained. The fabric is then cut to desired size for application.

## Description

The invention relates to a non-woven fabric to be used primarily as a non-adherent absorbent medical and veterinary dressing for application to all exudating and transudating wounds, burns, abscesses and to sprains in both human beings and animals and to a method of making it.

At present so-called non-adherent absorbent dressings are usually made of gauze and porous natural fibre such as cotton, rayon, or a combination of porous natural fibres with polyester.

These porous fibres absorb moisture or fluid into itself leading rapidly to the onset of what is known as "strike through" (i.e. fluid passing directly through the material). Also, despite claims to the contrary these materials to a greater or lesser extent do adhere to an exudating or transudating wound. At the onset of "strike through" the dressing requires to be changed. If it has adhered to the wound on removal it will inevitably reopen the wound or damage the regrowth of skin cells thus delaying the healing process. Traditional dressings such as the tulle gras dressing described in British Pharmacopoeia (1980), page 915, generally consist of a layer of gauze coated with paraffin wax. Such dressings have a number of desirable properties, and for this reason have been used extensively for many years, Among these advantages are their high degree of conformability and deformability, and the fact that their tackiness makes them very easy to apply. That is to say, a tulle gras dressing applied to a wound will usually remain in place simply by adhesion of the paraffin was to the patient's skin (or to itself in the case of a dressing wrapped around a finger, for example) while a securing bandage is applied. Tulle gras dressings are also quite inexpensive. However, tulle gras dressings do have a number of disadvantages. Principle amongst these is that, although initially non-adherent, they often become "dry" (in the sense of losing their paraffin coating) and consequently adhere to the wound to which they are applied. This effect is due to the paraffin coating becoming mobile at body temperatures and migrating into the wound or being absorbed into the backing of the dressing or bandage. In some cases, removal of a tulle gras dressing which has become dry in this way can cause considerable trauma. Indeed, it is quite common to have to soak tulle gras dressings in order to remove them. If tulle gras dressings are changed more frequently, in an attempt to avoid them becoming attached to the wound, this may delay wound healing and adds to nursing costs.

A further disadvantage of traditional tulle grass dressings is that fibres from the gauze may become incorporated in the wound, as may the paraffin coating of the dressing. Some authorities see the migration of paraffin into a wound as an undesirable effect and any paraffin found in a wound can be difficult to remove with normal aqueous wound cleansing agents. Moreover the pores of the gauze may become occluded if the paraffin wax coating is too heavy or as a result of the mobility of paraffin during use of the dressing. While occlusive dressings are appropriate in some circumstances. It is undesirable that the nursing staff should have no control over whether the dressing used is in fact occlusive.

Further disadvantages of conventional tulle gras dressings are that they are effectively opaque and somewhat unsightly appearance, and the paraffin can run during storage, making them particularly messy to apply.

The purpose of the present invention is to eliminate or significantly reduce the aforementioned drawbacks of traditional natural fibre dressings.

Processes are already in existence for the fabrication of non-woven textile products by needle-punching batts of fibres on a needleloom. The needle loom comprises a large number of needles which are intended to be passed through the fibre batt so that the needles each time pull a number of fibres with them finally producing an entanglement and interlocking of fibres.

With the foregoing in view a novel process for the fabrication of a non-woven fabric suitable for use as absorbent non-adherent medical and veterinary dressing is provided, which in the preferred embodiment comprises: the fabrication of two fibre batts on a needleloom each batt being made of 100% polyester fibre, 1.2 to 1.5 denier and being semi-dull, hollow or solid and being approximately 300 grammes per square metre in weight: the fabrication of a tightly woven "screen" made of a combination of cotton and polyester fibres. Typically the "screen" is made of woven fabric of 80% polyester and 20% cotton yarns. The construction is about 120 to 150 threads per square inch. The yarn count is around 32 to 40. Typically the "screen" is approximately 100 grammes per square metre in weight. The two fibre batts are then needle-punched by use of the needle loom one onto each side of the screen, thus making one fibre batt with a middle screen which will weigh approximately 700 grammes per square metre. Next the fibre batt thus made is subjected to needle-punching on the needleloom which may be of any known type, either once or several times on one side and once or several times on the other side such several times being repeated depending on the thickness or effect one wishes to obtain. The average thickness of the completed fibre batt required is 3 to 5 millimetres. Thereafter the non-woven fabric thus created is cut to the desired size (for example 20 centimetres by 20 centimetres) for use as medical or veterinary dressings as aforesaid.

The non-woven fabric thus made is able to transport, lift and hold fluid or moisture by means of capillary action by using non-porous fibres. The aforementioned screen is a preventive of the medical term "strike through" by encouraging the moisture or fluid to move across the fabric rather than straight through the fabric. The present invention is particulary suitable for use as wound dressings. It will be appreciated that other applications do exist. For example, it is useful in providing a suitable material for use as baby napkins and sanitary devices for use by women during menstruation. It is suitable for the prevention and treatment of pressure sores in bed-ridden patients.

The invention therefore provides, in one of its aspects, a method of producing non-woven fabric products able to transport lift and hold fluid or moisture by means of capillary action by using non-porous fibres, comprising the fabrication of two fibre batts on a needle loom, the fabrication of a tightly woven "screen" composed of polyester and cotton fibres and the bringing together of the needle-punched fibre batts on each side of the "screen" such that the "screen" forms an inner screen in the centre of the fabric thus created, the further needle-punching on the needle loom of the composite non-woven fabric thus formed on both sides so as to produce a fabric which has an average thickness of 3 to 5 millimetres. The invention provides in a further aspect a non-woven fabric product produced by the above method, having regard to the claims of the product.

## Claims

1. Process for the fabrication of non-woven fabric products able to transport lift and hold fluid or moisture by means of capillary action by using non-porous fibres characterized by the fact that it comprises the fabrication of two fibre batts on a needleloom; the fabrication of a tightly woven "screen" composed of polyester and cotton fibres and the bringing together of the needle-punched fibre batts on each side of the "screen" such that the "screen" forms an inner screen in the centre of the fabric thus created; the further needle-punching on the needleloom of the composite non-woven fabric thus formed on both sides so as to produce a fabric which has an average thickness of 3 to 5 millimetres.

2. Non-woven fabric products obtained by the application of the process according to claim 1.
